# EUROPEAN PATENT APPLICATION

(11) **EP 4 778 495 A1**
(43) Date of publication of application: **22.07.2026**
(21) Application number: 25152081.3
(22) Date of filing: 15.01.2025
(51) Int. Cl.: A61F 2/24

(54) **STENT DEVICE, CATHETER DEVICE, AND STENT SYSTEM**

(71) Applicant: CV Cardiovascular GmbH, 1190 Wien (AT)
(72) Inventor: EINHELLIG, Siegfried, 11990 Vienna (AT); AGRELI, Guilherme, 15-500 Sao Paulo (AT)
(74) Representative: Manitz Finsterwald Patent- und Rechtsanwaltspartnerschaft mbB

(57) **Abstract**

A stent device for a heart valve is provided, comprising a valve formed from and/or comprising biological tissue and/or polymeric material and an expandable or self-expanding metallic stent frame configured to support the valve, wherein the stent frame comprises protruding elements and anchor elements at one end thereof, wherein each of the anchor elements is configured to, in its open state, anchor in a base region of a heart valve leaflet when the stent device is deployed, wherein the anchor elements each comprise a first section directly adjacent to a first connecting point of the respective anchor element to the one end of the stent frame, wherein the first section is U-shaped, arch-shaped, and/or petal-shaped, wherein a curvature of the first section bends away from the stent frame, wherein preferably a plane of the curvature of the first section is orthogonal to a tangential of a circumference of the stent frame at the first connecting point.

## Description

The present invention relates to a stent device for a heart valve, a catheter device for deploying the stent device and a stent system.

Conventional valve replacement surgeries present a high risk for patients with severe heart valve diseases, such as insufficiency or stenosis. Stent devices that can be implanted via catherization can provide a minimally invasive solution. There is a need for stent devices that offer a precise control during the insertion procedure and a better adaption to the patient's anatomy.

It is therefore the object of the present invention to provide a stent device that better adapts to the patient's anatomy and can be more precisely positioned by a catheter device.

This object is satisfied by a stent device for a heart valve as defined in claim 1. The stent device of claim 1 comprises a valve formed from and/or comprising biological tissue and/or polymeric material and an expandable or self-expanding metallic stent frame configured to support the valve, wherein the stent frame comprises protruding elements and anchor elements at one end thereof, wherein each of the anchor elements is configured to, in its open state, anchor in a base region of a heart valve leaflet when the stent device is deployed, wherein the anchor elements each comprise a first section directly adjacent to a first connecting point of the respective anchor element to the one end of the stent frame, wherein the first section is U-shaped, arch-shaped, and/or petal-shaped, wherein a curvature of the first section bends away from the stent frame, wherein preferably a plane of the curvature of the first section is orthogonal to a tangential of a circumference of the stent frame at the first connecting point.

The stent frame can comprise self-expanding or balloon-expandable material, such as nitinol. Such material provides support to the valve and facilitates the stent frame's anchoring within the patient's cardiac anatomy. Since the curvature of the first sections of the U-shaped, arch-shaped, and/or petal-shaped anchor elements of the stent frame bends away from the stent frame, the anchor elements of the stent frame can better match the heart valve leaflets and enable a more precise positioning of the stent device during the implantation procedure. In other words, each first section extends in parallel to the radius of the stent frame, i.e. extends radially outward from the stent frame and away from the protruding elements.

Preferably, the plane of the curvature of the first section of each anchor element is orthogonal to the tangential of a circumference of the stent frame at the first connecting point. This further improves the adaption to the patient's anatomy and the deployment precision. The term orthogonal as used herein may generally refer to being approximately orthogonal including a tolerance and/or the positioning at an angle in the range of 40 to 50 degrees.

Preferably, between two and six of such anchor elements are provided, wherein most preferably three anchor elements are provided.

The stent device may be mountable to a flexible and articulated catheter device that can provide precise maneuvering during the implantation procedure. In this regard, the protruding elements of the stent frame can provide an interlock mechanism with the catheter device for precise control and release during the implantation procedure.

According to an embodiment, the anchor elements each comprise a second section, wherein the second section is arch-shaped, U-shaped, and/or petal-shaped, and preferably U-shaped. This can be particularly helpful for anchoring and adaption of the anchor elements to the heart valve leaflets.

According to a further embodiment, a plane of a curvature of the second section is orthogonal to the plane of the curvature of the first section. This can be particularly helpful for anchoring and adaption of the anchor elements to the heart valve leaflets.

According to a further embodiment, a distance of each of the anchor elements (in its open state), in particular the second section of each of the anchor elements, to the other end of the stent frame is in the range of 2 to 5 mm, preferably 3 to 4 mm. This can be particularly helpful for anchoring and adaption of the anchor elements to the heart valve leaflets.

Preferably, the curvature of the first section comprises an arc segment with a central angle of 150 to 180 degree, preferably 160 to 170 degrees. This may further improve the anchoring and adaption of the anchor elements to the heart valve leaflets.

According to a further embodiment, the protruding elements and the anchor elements are positioned alternatingly along the circumference of the stent frame.

Preferably, the anchor elements each comprise a third section directly adjacent to a second connecting point of the respective anchor element to the one end of the stent frame, wherein the third section is arch-shaped, U-shaped, and/or petal-shaped, wherein a curvature of the third section bends away from the stent frame, wherein preferably a plane of the curvature of the third section is orthogonal to a tangential to the circumference of the stent frame at the second connecting point, wherein preferably the plane of the curvature of the third section is orthogonal to the plane of the curvature of the second section, wherein preferably a ratio of a radius of the curvature of the third section to the radius of the curvature of the first section is in the range of 0.9 to 1.1, preferably 0.95 to 1.05, and more preferably 0.99 to 1.01. In other words, the third section may be similar to the first section or "mirror" the first section.

According to a further embodiment, the stent frame has a tubular shape in particular with a central axis.

According to a further embodiment, a ratio of the radius of the curvature of the first section of each of the anchor elements to a radius of the stent frame is in the range of 0.05 to 0.3, preferably 0.1 to 0.2. This can be particularly helpful for anchoring and adaption of the anchor elements to the heart valve leaflets.

For example, the radius of the stent frame can be in the range of 10 mm to 18 mm. In another example, the radius of curvature of the first section of each of the anchor elements can be in the range of 1 mm to 3 mm, and can be preferably 2 mm.

Preferably, the first section of each of the anchor elements comprises a first, second and third subsection, wherein the first subsection extends in a direction in parallel to the central axis of the stent frame, and wherein the second and third subsection form the curvature of the first section.

Preferably, the first section of each of the anchor elements has an inverted U-shape with respect to the protruding elements, the second section of each of the anchor elements is U-shaped with respect to the protruding elements, and/or the third section of each of the anchor elements has an inverted U-shape with respect to the protruding elements.

According to a further embodiment, each of the protruding elements comprises an end cap, wherein the end caps are positioned at the apices of the respective protruding element. Preferably, the end caps are oval-shaped or mushroom-shaped.

The end caps, and in particular the mushroom-shaped end caps, enable a secure yet releasable connection or interlock of the stent device to the catheter device. The end caps ensure sufficient fixation to push or pull the stent device within a holding means (such as a capsule) of the catheter device such that the stent device can be retracted into the holding means if needed. When fully released from the holding means, the protruding elements with the (preferably mushroom-shaped) end caps are fully deployed. Thereby, a safe release of the stent device and a safe removal of the catheter device can be ensured. Preferably, between two and six of such protruding elements are provided, wherein preferably three protruding elements are provided.

Preferably, the end caps comprise or are mushroom-heads.

Additionally or alternatively, the end caps preferably comprise or are eye-lets.

Preferably, the stent frame comprises three rows of cells, wherein preferably the cells are diamond-shaped.

Preferably, the shape of each of the anchor elements, in particular the U-shape, encompasses at least one row of cells, preferably at least two rows of cells.

Preferably, each protruding element comprises at least one cell at its base.

Preferably, the base of each of the protruding elements has a triangular shape.

According to a further embodiment, the stent device further comprises at least one sealing layer, wherein the sealing layer at least partly surrounds the other end of the stent frame and/or the valve.

According to a further embodiment, the sealing layer comprises a radial force and/or anti-leakage coating.

According to a further embodiment, the stent device further comprises a coating comprising a pharmaceutical drug to prevent post-operative complications. The drug may be selected from a group including antiplatelets, anticoagulants, antithrombotics, antibiotics and combinations thereof.

In another aspect, a catheter device for deploying a stent device as described herein is provided. The catheter device comprises a tubular body, wherein the tubular body comprises a multitude of rotatable, steerable and/or flexible sections along its longitudinal axis, wherein at least two sections of the multitude of sections differ in at least one parameter associated with deflection and/or elongation. The catheter device further comprises a holding means arranged at a terminal position of the tubular body, wherein the holding means is configured to releasably hold the stent device, wherein the holding means is configured to controllably release selected protruding elements of the stent device.

Preferably, the holding means comprises retaining elements complementary to the protruding elements of the stent device. For example, the holding means may comprise wires wrapped around the protruding elements.

Preferably, the parameter comprises an elastic modulus, shear modulus, and/or bulk modulus. Preferably, the parameter comprises an elastic modulus in x-, y-, and/or z-direction, wherein the x-and y-direction are perpendicular to the longitudinal axis of the tubular body and the z-direction is in parallel to the longitudinal axis of the tubular body.

According to an embodiment, at least one first section of the multitude of sections is steerable in a direction in parallel to the longitudinal axis of the tubular body, at least one second section of the multitude of sections is steerable in a perpendicular to the longitudinal axis of the tubular body, and at least one third section of the multitude of sections is configured to provide torque.

The steerability can be achieved, for example, by applying tension to a (steel) cable or wire, wherein one end thereof is connected to the distal end of the section that is to bend or be bend. The other end of the cable or wire can be attached to a handle that controls the bending. The provision of torque can be realized by rotating the handles of the delivery system.

Preferably the first section, second section, and third section are arranged alternatingly along the longitudinal axis of the tubular body
Preferably, at least two sections of the plurality of sections are manufactured by different manufacturing techniques. For example, the steerable tubes with good torque transmission are manufactured using a combination of laser-cutting techniques applied to metallic hypotubes (stainless steel, nitinol, cobalt-chromium) and coatings made of polymers with varying hardness and elasticity (such as TPU, PEBAX, HDPE, UHDPE, Nylon, PTFE, Carbosil). The production process for each tube can involve different laser-cut patterns and coatings with one or more polymers, allowing mechanical properties to be finely tuned to meet specific requirements.

Preferably, the first section, second section, and third section are positioned directly adjacent to the holding means.

Preferably, one to three first sections, one to three second sections, and one to three third sections are provided.

According to a further embodiment, the catheter device further comprises at least one radiopaque marker. This way, the catheter device can be precisely located during the implantation procedure.

According to a further embodiment, the catheter device is rotatable and/or able to rotate. For example, the catheter device is rotatable and/or able to rotate by actuation of the handle.

According to a further embodiment, the catheter devices is partially rotatable and/or able to partially rotate even when the stent device is partially deployed.

In this connection it should be noted that the catheter device may be constructed with a series of coaxial tubes, by way of example between three and five such tubes.

The tubes may each have a metallic structure optionally covered by multiple layers of a covering system, such as multiple layers of polymers. Such a construction pattern of this structure, optionally together with the covering system, enables torque transmission and thus allows the device to rotate even when the valve (or stent) is partially released.

By way of example such a catheter tube, i.e. its structure and the selection of the material, is designed for torque transmission, ensuring that rotation is conveyed throughout the entire system.

Metallic tubes can be selected, by way of example tubes made of stainless steel or special alloys, as these are not only capable of withstanding torsional stresses, but can also be sterilized in a very convenient manner.

Multiple polymeric layers can be added, as they confer low friction tubes, protection against mechanical damage, and also anti-thrombogenic properties
If a plurality of tubes are used these may require a coupling system between the tubes.

Such coupling systems may comprise one or more internal fittings that ensure all tubes rotate simultaneously and uniformly when torque is applied at a handle or the proximal end of the device.

This type of coupling may be distinct as it must allow rotation without misalignment or locking, even when the valve is partially deployed.

In this connection it should be noted that state-of-the-art catheter devices cannot efficiently transmit torque when the valve or stent is partially deployed, as part of the system may become locked or blocked.

In such conventional systems, rotation is generally only effective when the stent/valve is either fully confined within the introducer or completely released.

In contrast, to such prior art devices, the coaxial design and choice of materials of the catheter device, particularly in the design and composition of the tubes and their couplings, enables continuous torque transmission at any stage of device deployment.

According to a further embodiment, the catheter device is bendable and/or able to bend in four distinct directions. This may be achieved, for example, by multiple wires attached to the distal ends of the sections at certain positions along their circumference.

In another aspect, a stent system is provided comprising a stent device as described herein and a catheter device as described herein.

According to an embodiment, the stent device is retained by the catheter device.

The disclosure with respect to the stent device is also valid for the catheter device and the stent system. This is particularly true for embodiments and advantages.

Further, it is to be understood that all features and embodiments disclosed herein can be combined if not explicitly stated otherwise.

Various features and advantages of the present invention will become more apparent from the following exemplary description and the examples shown in the accompanying drawings, wherein:
- Fig. 1: shows a perspective view of a stent device;
- Fig. 2: shows a side view of the stent device of Fig. 1;
- Fig. 3: shows a perspective view of the stent frame of the stent device of Fig. 1;
- Fig. 4: shows a side view of the stent frame of Fig. 1;
- Fig. 5: shows a cut out of Fig. 3;
- Fig. 6: shows a cut out of Fig. 3;
- Fig. 7: schematically shows a top view of the cut out of Fig. 5;
- Fig. 8: schematically shows a catheter device;
- Fig. 9: schematically shows a stent system;
- Fig. 10: schematically shows a stent system.

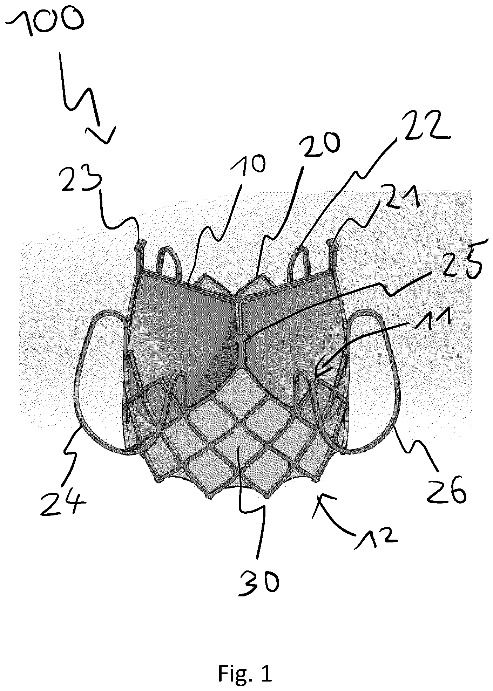

Fig. 1 schematically shows a perspective view of a stent device 100 comprising a valve 10 formed from bovine pericardium and an expandable or self-expanding metallic stent frame 20 configured to support the valve 10. The stent frame 20 comprises three protruding elements 21, 23, 25 and three anchor elements 22, 24, 26 at one end 11 thereof, wherein each of the anchor elements 22, 24, 26 is configured to, in its open state, anchor in a base region of a heart valve leaflet (not shown) when the stent device 100 is deployed. As shown in Fig. 1, the anchor elements 22, 24, 26 are U-shaped with respect to the protruding elements 21, 23, 25. The protruding elements 21, 23, 25 and the anchor elements 22, 24, 26 are positioned alternatingly along the circumference of the stent frame 20. It is to be understood that also more or less than three anchor elements 22, 24, 26 and more or less than three protruding elements 21, 23, 25 may be provided.

The stent device as shown in Fig. 1 further comprises a sealing layer 30, wherein the sealing layer 30 (completely) surrounds the other end 12 of the stent frame 20.

The stent device 100 further comprises coatings (not shown in Fig. 1) comprising a pharmaceutical drug to prevent post-operative complications. The coatings can be applied to the valve and/or the stent frame as follows. A solution is prepared by dissolving a degradable polymer such as Poly(D,L-lactide), Poly (D,L lactide-co-glycolide) or Poly (L-lactide-co-glycolide) in a volatile solvent such as chloroform, acetone, or dichloromethane at a concentration from 0,5 up to 6,7% (m/v) at room temperature. For example, 10 g of Poly(D,L-lactide), PDLLA, is dissolved in 150 mL of chloroform. The solution is sterile-filtered (pore size of 0.2 µm). A sterile solution of an anticoagulant such as Apixaban then mixed into the PDLLA/chloroform solution at a concentration of 1 mg of Apixaban per mL of the PDLLA/chloroform solution. It has been found that this amount provides an effective balance between drug release kinetics and the mechanical stability of the coating, ensuring adequate pharmacological action while preserving the stent's structural integrity.

For instance, if you have prepared 150 mL of the PDLLA/chloroform solution (as in the example of dissolving 10 g of PDLLA in 150 mL of chloroform), you would add 150 mg of Apixaban. This mixture is then stirred thoroughly to achieve a uniform solution before coating the valve or the stent frame.

The resulting mixture is loaded into an airbrush and, at best, used immediately to coat the valve and/or the stent frame. In case the coatings are applied to valve before its attachment to the stent frame, the valve can be temporarily placed into a holder. The solvent is evaporated using an air dryer. A coating gel is prepared by adding a solvent such as 99% ethanol to PDLLA powder, followed by ultrapure water in a 6:4 ratio (based on the weight for the powders and the volume of the liquids).

The gel is loaded with antibiotics at 5X MIC (e.g., Vancomycin, Cubicin). Additionally or alternatively, glycosaminoglycan such as heparin is added at a concentration of 5 mg/mL. This mixture is then loaded in an airbrush and sprayed at a distance of between 1 to 50 cm to achieve a coating of a thickness of 1 to 20 µm, preferably of 5 to 10 µm. This range strikes a balance between adequate drug loading, the mechanical stability of the polymer, and the desired drug release profile-without significantly increasing the overall device profile or compromising the stent's flexibility.

The solvent is evaporated using an air dryer. A permanent polymer such as Carbosil is dissolved in DMSO at a concentration between 10% to 25% w/v, preferably 25% w/v (weight/volume). The valve (attached to the holder) and/or the stent frame is immersed in the Carbosil/DMSO solution, then pulled out, and allowed to dry for 10 minutes to facilitate the removal of excess polymer solution by gravity. Subsequently, the solvent is evaporated using an air dryer. Additional coatings comprising a drug selected from a group including antiplatelets, anticoagulants, antithrombotics, antibiotics (e.g. Cubicin, Vancomycin and Gentamicin) and combinations thereof can be applied as described above.

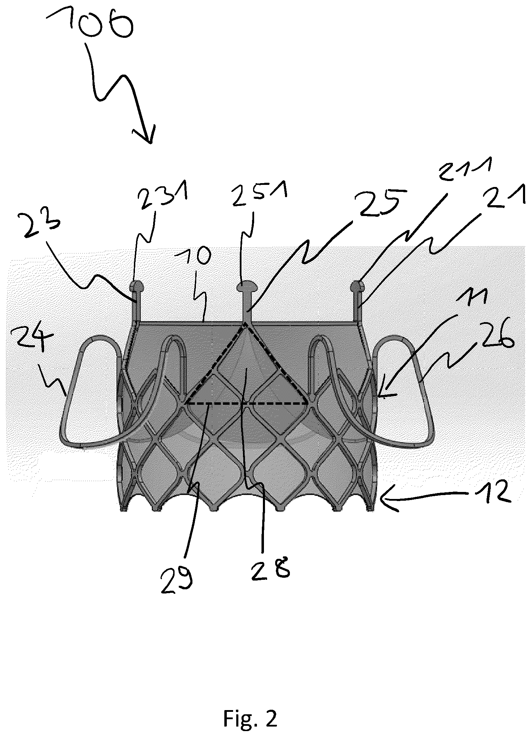

Fig. 2 schematically shows a side view of the stent device of Fig. 1. Each of the protruding elements 21, 23, 25 comprises a mushroom-shaped end cap 211, 231, 251, wherein the end caps are positioned at the apices of the respective protruding element 21, 23, 25. The stent frame 20 comprises three rows of cells 28, wherein the cells 28 are diamond-shaped. Each protruding element 21, 23, 25 comprises at least one (full) cell 28 at its base 29. As indicated in Fig. 2, the base 29 of each of the protruding elements has a triangular shape.

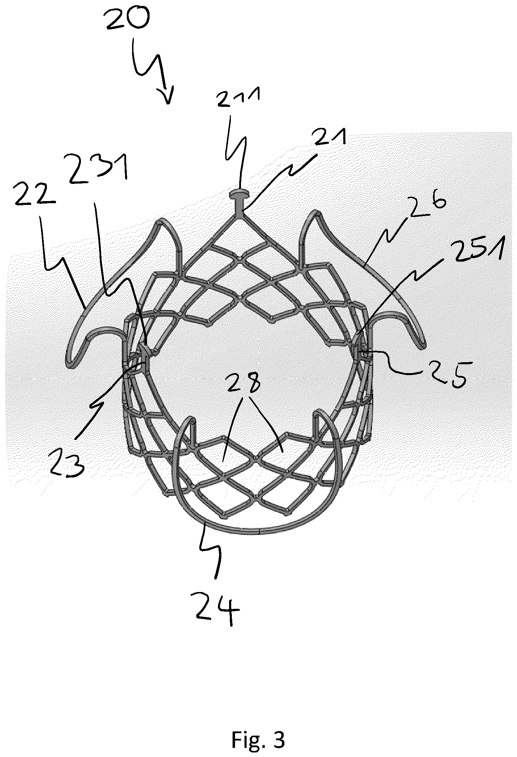

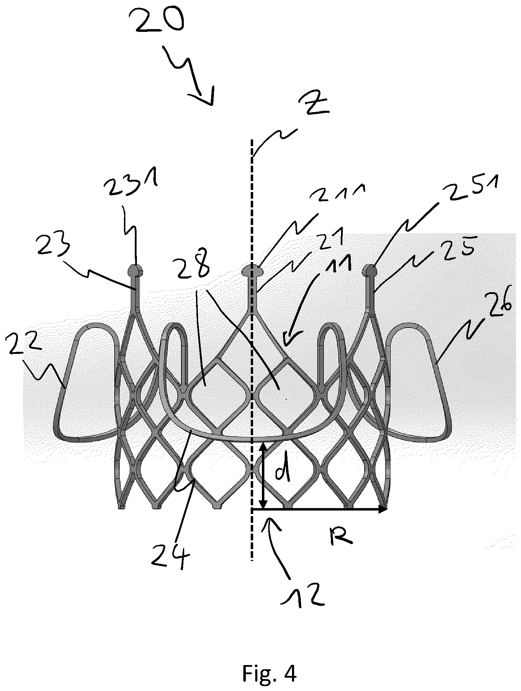

Fig. 3 and Fig. 4 show a perspective view and a side view, respectively, of the stent frame 20 of the stent device 100 of Fig. 1. The U-shape of each of the anchor elements 22, 24, 26 encompasses at least two cells 28 along the circumference of the stent frame 20. As indicated in Fig. 4, The stent frame 20 has a tubular shape with a central axis Z and radius R. The U-shape of each of the anchor elements 22, 24, 26 encompasses at least one row of cells 28 along the longitudinal direction of the stent frame 20, i.e. along the central axis Z. As indicated in Fig. 4, the distance d of each of the anchor elements 22, 24, 26 to the other end 12 of the stent frame (when the stent device is fully deployed) is in the range of 2 to 5 mm, preferably 3 to 4 mm.

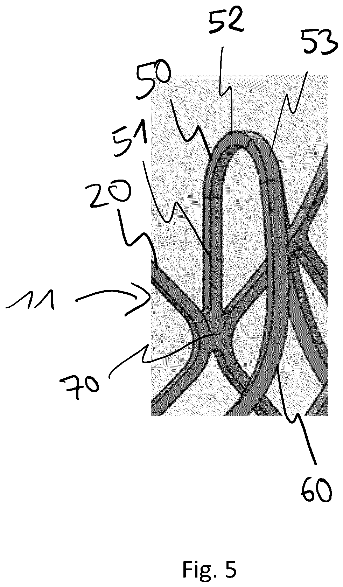

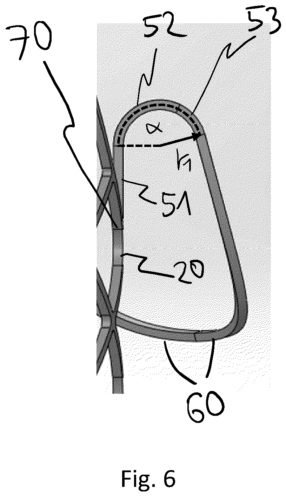

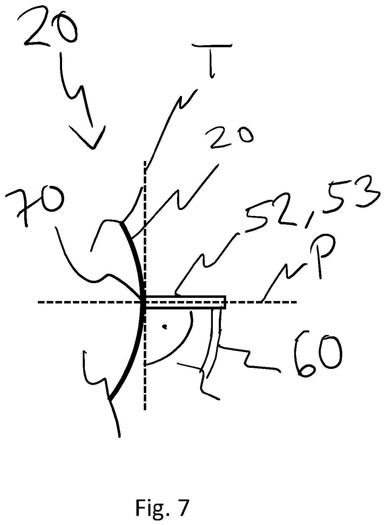

Fig. 5 and Fig. 6 each show a cut out of Fig. 3. Fig. 7 schematically shows a top view of the cut out of Fig. 5. The anchor elements 22, 24, 26 each comprise a first section 50 directly adjacent to a first connecting point 70 of the respective anchor element 22, 24, 26 to the one end 11 of the stent frame 20. A curvature of the first section 50 bends away from the stent frame 20, wherein a plane P (Fig. 7) of the curvature of the first section 50 is orthogonal to a tangential T (Fig. 7) of a circumference of the stent frame 20 at the first connecting point 70. The first section 50 comprises a first subsection 51 (Fig. 5 and Fig. 6, not shown in Fig 7), a second subsection 52 (Fig. 5 to Fig. 7), and a third subsection 53 (Fig. 5 to Fig. 7), wherein the first subsection 51 extends in a direction in parallel to the central axis Z (not shown in Fig. 5 and 6) of the stent frame 20, and wherein the second and third subsection 52, 53 form the curvature of the first section 50.

The first section 50 of each of the anchor elements 22, 24, 26 has an inverted U-shape with respect to the protruding elements 21, 23, 25 and the second section 60 of each of the anchor elements 22, 24, 26 is U-shaped with respect to the protruding elements 21, 23, 25. Each of the anchor elements 22, 24, 26 further comprises a third section (not shown in Fig. 5 to Fig. 7), which is directly adjacent to a second connecting point (not shown in Fig. 5 to Fig. 7) of the respective anchor element 22, 24, 26 to the one end 11 of the stent frame 20. The third section is similar to the first section 50 or "mirrors" the first section 50.

The radius r₁ (Fig. 6) of curvature of the first section 50 of each of the anchor elements 22, 24, 26 is in the range of 1 mm to 3 mm, and is preferably 2 mm. The curvature of the first section 50 comprises an arc segment with a central angle α of 150 to 180 degree, preferably 160 to 170 degrees.

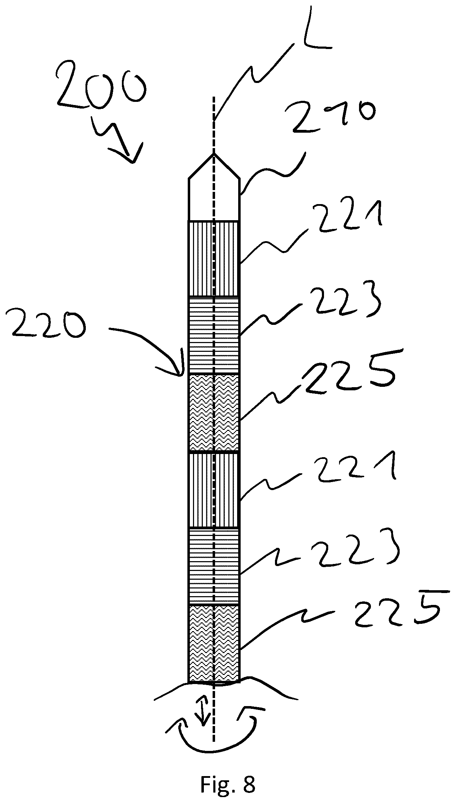

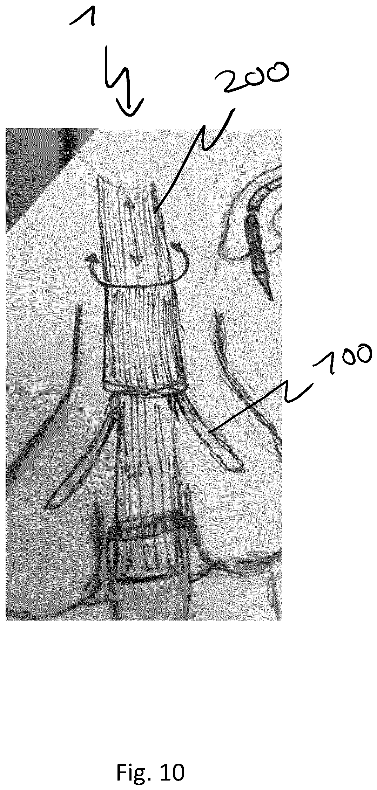

Fig.8 schematically shows a catheter device 200 for deploying the stent device 100 as shown in Fig. 1. The catheter device 200 comprises a tubular body 220, wherein the tubular body 220 comprises a multitude of rotatable, steerable and/or flexible sections 221, 223, 225 along its longitudinal axis L. The sections 221, 223, 225 differ in at least one parameter associated with deflection and/or elongation, wherein the parameter comprises an elastic modulus, shear modulus, and/or bulk modulus. A holding means 210, here a capsule, is arranged at a terminal position of the tubular body 220, wherein the holding means 210 is configured to releasably hold the stent device 100 (not shown in Fig. 8). At least one first section 221 of the multitude of sections is steerable in a direction in parallel to the longitudinal axis L of the tubular body 220, at least one second section 223 of the multitude of sections is steerable in a direction perpendicular to the longitudinal axis L of the tubular body 220, and at least one third section 225 of the multitude of sections is configured to provide torque. As shown in Fig. 8, the first section 221, second section 223, and third section 225 are arranged alternatingly along the longitudinal axis L of the tubular body 220. The first section 221, second section 223, and third section 225 are positioned directly adjacent to the holding means 210. Moreover, two first sections 221, two second sections 223, and two third sections 225 are provided. It is to be understood that also more or less than two first sections 221, two second sections 223, and two third sections 225 may be provided. The catheter device 200 is rotatable and/or able to rotate (as indicated in Fig. 8 and Fig. 10). The catheter device 200 is bendable and/or able to bend in four distinct directions.

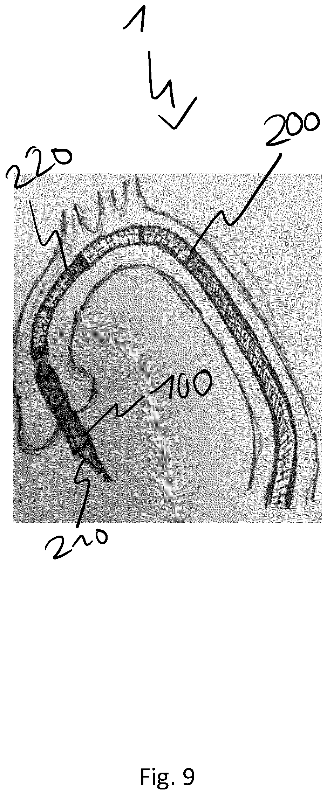

Fig. 9 and Fig.10 each show schematically a stent system 1 comprising a stent device 100 (Fig. 1 to Fig. 8) and catheter device 200 (Fig. 8). The stent device 100 can be contained within the holding means 210 of the catheter device 200 and can be released in two stages. A proximal portion of the catheter device 200 with the holding means 210 is positioned in the left ventricular outflow tract (LVOT), while another portion is maintained in the ascending aorta/Sinus of Valsalva. The stent device 100 is connected to the catheter device 200 via the mushroom-shaped end caps 211, 231, 251 of the protruding elements 21, 23, 25 (Fig. 1 to Fig 4). Advancing the proximal portion with the holding means 210 partially releases the stent device 100 (Fig 10), while retracting the holding means 210 maintained in the aorta fully releases the stent device 100. The catheter devices 200 is partially rotatable and/or able to partially rotate even when the stent device 100 is partially deployed (Fig. 10).

### Reference numeral list

- 10: valve
- 11: one end of the stent frame
- 12: other end of the stent frame
- 20: stent frame
- 21: protruding element
- 22: anchor element
- 23: protruding element
- 24: anchor element
- 25: protruding element
- 26: anchor element
- 28: cell
- 29: base
- 30: sealing layer
- 50: first section of an anchor element
- 51: first subsection
- 52: second subsection
- 53: third subsection
- 60: second section of an anchor element
- 100: stent device
- 200: catheter device
- 211: end cap
- 221: first section of the tubular body
- 223: second section of the tubular body
- 225: third section of the tubular body
- 231: end cap
- 251: end cap

- α: central angle
- d: distance
- L: longitudinal axis
- P: plane of curvature
- R: radius of the stent frame
- r₁: radius of the curvature of the first subsection
- T: tangential
- Z: zentral axis

## Claims

1. A stent device (100) for a heart valve, comprising
a valve (10) formed from and/or comprising biological tissue and/or polymeric material; and
an expandable or self-expanding metallic stent frame (20) configured to support the valve (10), wherein the stent frame (20) comprises protruding elements (21, 23, 25) and anchor elements (22, 24, 26) at one end (11) thereof, wherein each of the anchor elements (22, 24, 26) is configured to, in its open state, anchor in a base region of a heart valve leaflet when the stent device (100) is deployed, wherein the anchor elements (22, 24, 26) each comprise a first section (50) directly adjacent to a first connecting point (70) of the respective anchor element (22, 24, 26) to the one end (11) of the stent frame (20), wherein the first section (50) is U-shaped, arch-shaped, and/or petal-shaped, wherein a curvature of the first section (50) bends away from the stent frame (20), wherein preferably a plane (P) of the curvature of the first section (50) is orthogonal to a tangential (T) of a circumference of the stent frame (20) at the first connecting point (70).

2. The stent device (100) of claim 1,
wherein the anchor elements (22, 24, 26) each comprise a second section (60), wherein the second section (60) is arch-shaped, U-shaped, and/or petal-shaped, wherein preferably a plane of a curvature of the second section (60) is orthogonal to the plane of the curvature of the first section (50); wherein a distance (d) of each of the anchor elements (22, 24, 26), in particular the second section (60) of each of the anchor elements (22, 24, 26), to the other end (12) of the stent frame (20) is in the range of 2 to 5 mm, preferably 3 to 4 mm; and/or
wherein the protruding elements (21, 23, 25) and the anchor elements (22, 24, 26) are positioned alternatingly along the circumference of the stent frame (20).

3. The stent device (100) of claims 1 or 2, wherein the stent frame (20) has a tubular shape in particular with a central axis (Z), wherein preferable a ratio of the radius (r₁) of the curvature of the first section (50) of each of the anchor elements (22, 24, 26) to a radius (R) of the stent frame (20) is in the range of 0,05 to 0,3, preferably 0,1 to 0,2.

4. The stent device (100) of any one of the preceding claims, wherein each of the protruding elements (21, 23, 25) comprises an end cap (211, 231, 251), wherein the end caps (211, 231, 251) are positioned at the apices of the respective protruding element (21, 23, 25), wherein preferably the end caps (211, 231, 251) are oval-shaped or mushroom-shaped.

5. The stent device (100) of any one of the preceding claims, further comprising at least one sealing layer (30), wherein the sealing layer (30) at least partly surrounds the other end (12) of the stent frame (20) and/or the valve (10).

6. The stent device (100) of claim 5, wherein the sealing layer (30) comprises a radial force and/or anti-leakage coating.

7. The stent device (100) of any one of the preceding claims, further comprising a coating comprising a pharmaceutical drug to prevent post-operative complications.

8. A catheter device (200) for deploying a stent device (100) according to any one of the preceding claims, the catheter device (200) comprising
a tubular body (220), wherein the tubular body (220) comprises a multitude of rotatable, steerable and/or flexible sections (221, 223, 225) along its longitudinal axis (L), wherein at least two sections of the multitude of sections (221, 223, 225) differ in at least one parameter associated with deflection and/or elongation, wherein preferably the parameter comprises an elastic modulus, shear modulus, and/or bulk modulus; and
a holding means (210) arranged at a terminal position of the tubular body (220), wherein the holding means (210) is configured to releasably hold the stent device (100).

9. The catheter device (200) of claim 8,
wherein at least one first section (221) of the multitude of sections is steerable in a direction perpendicular to the longitudinal axis (L) of the tubular body (220);
wherein at least one second section (223) of the multitude of sections is steerable in a direction in parallel to the longitudinal axis (L) of the tubular body (220); and
wherein at least one third section (225) of the multitude of sections is configured to provide torque, wherein preferably the first section (221), second section (223), and third section (225) are arranged alternatingly along the longitudinal axis (L) of the tubular body (220).

10. The catheter device (200) of claim 8 or 9, further comprising at least one radiopaque marker.

11. The catheter device (200) of any one of claims 8 to 10, wherein the catheter device (200) is rotatable and/or able to rotate.

12. The catheter device (200) of any one of claims 8 to 11, wherein the catheter devices (200) is partially rotatable and/or able to partially rotate even when the stent device (100) is partially deployed.

13. The catheter device (200) of any one of claims 8 to 12, wherein the catheter device (200) is bendable and/or able to bend in four distinct directions.

14. A stent system comprising a stent device (100) according to any one of claims 1 to 7 and a catheter device (200) according to any one of claims 8 to 13.

15. The stent system of claim 14, wherein the stent device (100) is retained by the catheter device (200).
